(19) Europäisches Patentamt
European Patent Office
Office européen des brevets

(11) Veröffentlichungsnummer: **0 467 905 B1**

# (12) EUROPÄISCHE PATENTSCHRIFT

(45) Veröffentlichungstag der Patentschrift: **07.09.94**

(51) Int. Cl.^5: **C07D 307/92**, //C11B9/00

(21) Anmeldenummer: **90905467.8**

(22) Anmeldetag: **06.04.90**

(86) Internationale Anmeldenummer:
**PCT/EP90/00541**

(87) Internationale Veröffentlichungsnummer:
**WO 90/12793 (01.11.90 90/25)**

## (54) VERFAHREN ZUR STEREOSELEKTIVEN HERSTELLUNG VON 8-g(a),12-OXIDO-13,14,15,16-TETRANORLABDAN.

(30) Priorität: **14.04.89 DE 3912318**

(43) Veröffentlichungstag der Anmeldung:
**29.01.92 Patentblatt 92/05**

(45) Bekanntmachung des Hinweises auf die Patenterteilung:
**07.09.94 Patentblatt 94/36**

(84) Benannte Vertragsstaaten:
**AT BE CH DE ES FR GB IT LI NL**

(56) Entgegenhaltungen:
**DE-C- 850 750**

**Chemical Abstracts, Band 105, Nr.15, 13.Oktober 1986, (Columbus, Ohio,US), siehe Seite 703, Zusammenfassung 134193k,**

(73) Patentinhaber: **Henkel Kommanditgesellschaft auf Aktien**

**D-40191 Düsseldorf (DE)**

(72) Erfinder: **BRUNS, Klaus**
**Notburgaweg 6**
**D-4150 Krefeld (DE)**
Erfinder: **STALBERG, Theo**
**Robert-Koch-Str. 43**
**D-4019 Monheim (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft ein Verfahren zur stereoselektiven Herstellung von 8α,12-Oxido-13,14,15,16-tetranorlabdan sowie die Verwendung von Aluminiumoxid bestimmter Eigenschaften zur stereoselektiven Herstellung von 8α,12-Oxido-13,14,15,16-tetranorlabdan.

8α,12-Oxido-13,14,15,16-tetranorlabdan 2 (Ambroxan$^{R}$) ist ein außerordentlich wertvoller Ambrariechstoff, der in der Ambra, einer Stoffwechselausscheidung des Pottwals, enthalten ist (Ullmanns Encyklopädie der technischen Chemie, Band 20, Seite 283, Verlag Chemie Weinheim 1981). Synthetisch kann Ambroxan$^{R}$ aus Sclareol durch oxidativen Seitenkettenabbau und nachfolgende Reduktion des gebildeten Lactons (Sclareolid) gemäß US 30 50 532 hergestellt werden. Die Überführung von Sclareolid in das geruchlose Diol 8α,12-Dihydroxy-13,14,15,16tetranorlabdan 1 erfolgt in an sich bekannter Weise, beispielsweise durch Reduktion mit Lithiumaluminiumhydrid (Helv. Chim. Acta 33, 1310 (1950)), mit Natriumborhydrid (Chem. Abstr. 57, 7316a) oder mit Kaliumborhydrid/Lithiumchlorid-Mischungen (Chem. Abstr. 94, 15913q).

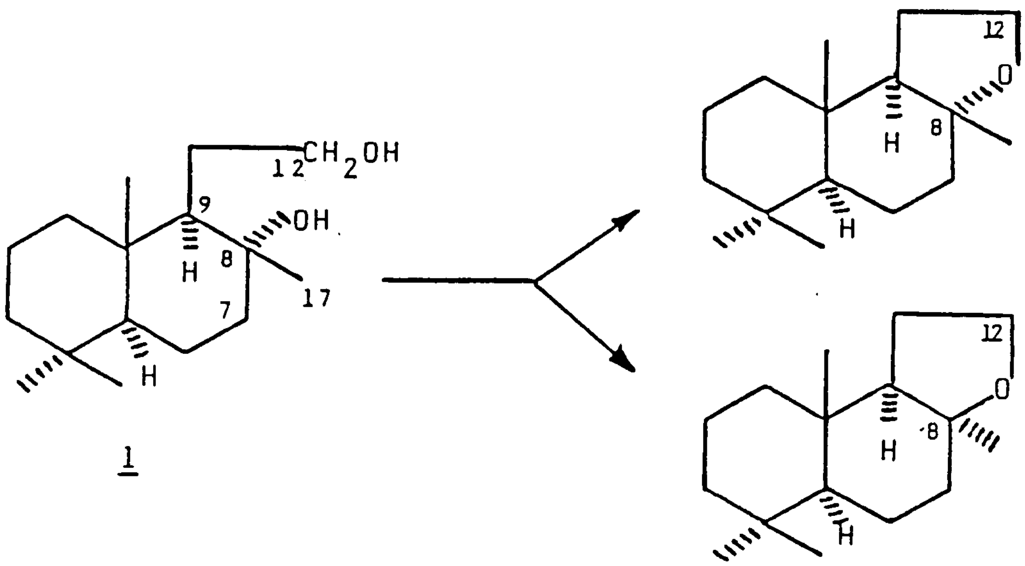

1

2

2a

Die Dehydratisierung von Diol 1 bei gleichzeitigem Ringschluß zu Ambroxan$^{R}$ kann mit sauren Katalysatoren, beispielsweise p-Toluolsulfonsäure, p-Toluolsulfonsäurechlorid, katalytischen Mengen an Schwefelsäure sowie sauren Ionenaustauschern in verschiedenen Lösungsmitteln, beispielsweise Toluol, Hexan, Pyridin, Tetrahydrofuran oder Methanol, vorzugsweise bei Siedetemperatur, durchgeführt werden. Bei diesen Verfahren werden häufig Gemische erhalten, die neben ungesättigten Alkoholen, Olefinen sowie dem nahezu geruchlosen 8-epi-Oxid 2a (Helv. Chim. Acta 68, 2022 (1985)) das geruchsaktive, äquatorial 8α,12-Oxido-verknüpfte Furan 2 lediglich in Mengen zwischen 20 und 50 Gew.-% enthalten.

In US 3 029 255 wird die Verwendung von β-Naphthalinsulfonsäure oder Tonerde als Dehydratisierungskatalysatoren bei der Herstellung von Ambroxan$^{R}$ beschrieben. Bei diesem Verfahren entstehen neben Verharzungsprodukten und Olfinen weitere Nebenprodukte, so daß die Ausbeuten an Ambroxan$^{R}$ bei weniger als 77 % liegen.

Aus JP 61/33184, zitiert in Chem. Abstr. 105, 134193k (1986), ist bekannt, daß bei Verwendung von aktiver Weißerde, Tonerde oder Kieselerde, die mit 1 bis 20 Gew.-% Schwefelsäure, Phosphorsäure oder Polyphosphorsäure beladen sind, die theoretischen Ausbeuten an Ambroxan$^{R}$ auf 85 bis 90,5 % gesteigert werden können. In zwei Beispielen wird die Reinheit, d. h. der Gehalt an geruchsaktivem Furan 2, mit 97 % und 98 % angegeben.

Die Aufgabe der Erfindung bestand in der Entwicklung eines Verfahrens zur stereoselektiven Herstellung von 8α,12-Oxido-13,14,15,16-tetranorlabdan, mit dem es möglich ist, die Ausbeute an Ambroxan$^{R}$ mit einer Reinheit >95 % auf deutlich über 90 % zu steigern.

Überraschenderweise wurde gefunden, daß Ambroxan$^{R}$ mit einer Reinheit von >97 Gew.-% und in einer Ausbeute von >95 % hergestellt werden kann, wenn 8α,12-Dihydroxy-13,14,15,16-tetranorlabdan 1 mit Aluminiumoxid bestimmter Eigenschaften dehydratisiert wird.

Gegenstand der Erfindung ist dementsprechend ein Verfahren zur stereoselektiven Herstellung von 8α,12-Oxido-13,14,15,16-tetranorlabdan durch Dehydratisierung von 8α,12-Dihydroxy-13,14,15,16-tetranorlabdan 1 mit säurebeladenem Aluminiumoxid, welches dadurch gekennzeichnet ist, daß man 8α,12-Dihydroxy-13,14,15,16-tetranorlabdan mit - bezogen auf dieses 8α,12-Dihydroxy-13,14,15,16-tetranorlabdan - 60 bis 80 Gew.-% Aluminiumoxid mit einer Salzsäurebeladung zwischen 0,4 und 0,6 Gew.-% - bezogen auf Aluminiumoxid, einer Schüttdichte von 0,9 g/$cm^3$ und einem Kornbereich zwischen 0,05 und 0,2 mm (70 bis 290 mesh) dehydratisiert.

Erfindungsgegenstand ist ferner die Verwendung von Aluminiumoxid mit einer Salzsäurebeladung zwischen 0,4 und 0,6 Gew.-% - bezogen auf Aluminiumoxid -, einer Schüttdichte von 0,9 g/$cm^3$ und einem Kornbereich zwischen 0,05 und 0,2 mm (70 bis 290 mesh) zur stereoselektiven Herstellung von 8α,12-Oxido-13,14,15,16-tetranorlabdan aus 8α,12-Dihydroxy-13,14,15,16-tetranorlabdan.

Das Diol 1 mit einem Wassergehalt zwischen 0 und 1,5 Gew.-% wird vorzugsweise mit - bezogen auf Diol 1 - 70 bis 80 Gew.-% Aluminiumoxid mit den oben genannten Eigenschaften dehydratisiert. Die Dehydratisierung kann ohne Lösungsmittel oder vorzugsweise in Lösungsmitteln, beispielsweise in Toluol und/oder Xylol, durchgeführt werden. Die Reaktionstemperatur liegt vorzugsweise zwischen 120 und 140 °C. Das während der Dehydratisierung gebildete Wasser kann beispielsweise durch azeotrope Destillation aus dem Reaktionsgemisch entfernt werden. Nach beendeter Dehydratisierung wird das Reaktionsgemisch in an sich bekannter Weise aufgearbeitet.

Beispiel

Herstellung von 8α,12-Oxido-13,14,15,16-tetranorlabdan (2)

16,5 kg Diol 1, gelöst in 22 l Toluol, wurden in einer Stickstoffatmosphäre so lange am Wasserabscheider erhitzt bis sich kein Wasser mehr abtrennte. Anschließend wurde auf 100 °C abgekühlt und nach Zugabe von 12,5 kg Aluminiumoxid (Säurebeladung: 0,5 Gew.-% Salzsäure, Schüttdichte: 0,9 g/$cm^3$, Kornbereich: 0,05 bis 0,2 mm (70 bis 290 mesh)) in 6 Stunden 1,5 kg Reaktionswasser azeotrop entfernt.

Anschließend wurde das Reaktionsgemisch über eine Drucknutsche filtriert. Das abgesaugte Aluminiumoxid wurde in 7,5 l Toluol aufgeschlämmt und erneut filtriert. Die vereinigten organischen Phasen wurden nacheinander 2 mal mit je 5 l 1 Gew.-%iger wäßriger Salpetersäure, 2 mal mit je 4 l 1 Gew.-%iger Natronlauge und 1 mal mit 4 l 15 Gew.-%iger wäßriger Natriumsulfatlösung bei etwa 45 °C gewaschen. Anschließend wurde das Lösungsmittel abdestilliert und das erhaltene Rohprodukt bei 115 - 125 °C / 50 Pa destilliert und auf eine Schuppwalze überführt. Es wurden 14,5 g (theoretische Ausbeute: 95 %) 8α,12-Oxido-13,14,15,16-tetranorlabdan mit folgenden Eigenschaften erhalten:

Farblose, kristalline Schuppen, Schmelzpunkt: 76 bis 77 °C (Acetonitril) [77 bis 77,5 °C in Helv. Chim. Acta 68 2022 (1985)]

$$\alpha^{25}_{589} = -26{,}7\,° \text{ (Chloroform; Konzentration = 10,0 Gew.-\%)}$$

$$\alpha^{20}_{589} = -24{,}7\,° \text{ (Chloroform; Konzentration = 1,0 Gew.-\%)}$$

**Patentansprüche**

1. Verfahren zur stereoselektiven Herstellung von 8α,12-Oxido-13,14,15,16-tetranorlabdan durch Dehydratisierung von 8α,12-Dihydroxy-13,14,15,16-tetranorlabdan mit säurebeladenem Aluminiumoxid, dadurch gekennzeichnet, daß man 8α,12-Dihydroxy-13,14,15,16-tetranorlabdan mit - bezogen auf dieses 8α,12-Dihydroxy-13,14,15,16-tetranorlabdan - 60 bis 80 Gew.-% Aluminiumoxid mit einer Salzsäurebeladung zwischen 0,4 und 0,6 Gew.-% - bezogen auf Aluminiumoxid -, einer Schüttdichte von 0,9 g/$cm^3$ und einem Kornbereich zwischen 0,05 und 0,2 mm (70 bis 290 mesh) dehydratisiert.

2. Verfahren nach Anspruch 1, dadurch gekennzeichnet, daß man 8α,12-Dihydroxy-13,14,15,16-tetranorlabdan mit - bezogen auf dieses 8α,12-Dihydroxy-13,14,15,16-tetranorlabdan- 70 bis 80 Gew.-%

salzsäurebeladenem Aluminiumoxid dehydratisiert.

3. Verfahren nach einem oder beiden der Ansprüche 1 bis 2, dadurch gekennzeichnet, daß man die Dehydratisierung in Lösungsmitteln, vorzugsweise in Toluol und/oder Xylol, durchführt.

4. Verfahren nach einem oder mehreren der Ansprüche 1 bis 3, dadurch gekennzeichnet, daß man bei Temperaturen zwischen 120 und 140 °C dehydratisiert.

5. Verwendung von Aluminiumoxid mit einer Salzsäurebeladung zwischen 0,4 und 0,6 Gew.-% - bezogen auf Aluminiumoxid - einer Schüttdichte von 0,9 g/cm$^3$ und einem Kornbereich zwischen 0,05 und 0,2 mm (70 bis 290 mesh) zur stereoselektiven Herstellung von 8α,12-Oxido-13,14,15,16-tetranorlabdan aus 8α,12-Dihydroxy-13,14,15,16-tetranorlabdan.

## Claims

1. A process for the stereoselective preparation of 8α,12-oxido-13,14,15,16-tetranorlabdane by dehydration of 8α,12-dihydroxy-13,14,15,16-tetranorlabdane 1 with acid-charged aluminium oxide, characterized in that 8α,12-dihydroxy-13,14,15,16-tetranorlabdane 1 is dehydrated with - based on this 8α,12-dihydroxy-13,14,15,16-tetranorlabdane - 60 to 80% by weight of aluminium oxide charged with 0.4 to 0.6% by weight, based on aluminium oxide, of acid and having an apparent density of 0.9 g/cm$^3$ and a particle size range of 0.05 to 0.2 mm (70 to 290 mesh).

2. A process as claimed in claim 1, characterized in that 8α,12-dihydroxy-13,14,15,16-tetranorlabdane is dehydrated with - based on this 8α,12-dihydroxy-13,14,15,16-tetranorlabdane - 70 to 80% by weight of acid-charged aluminium oxide.

3. A process as claimed in one or both of claims 1 and 2, characterized in that the dehydration is carried out in solvents, preferably in toluene and/or xylene.

4. A process as claimed in one or more of claims 1 to 3, characterized in that dehydration is carried out at temperatures of 120 to 140 °C.

5. The use of aluminium oxide charged with 0.4 to 0.6% by weight, based on aluminium oxide, of acid and having an apparent density of 0.9 g/cm$^3$ and a particle size range of 0.05 to 0.2 mm (70 to 290 mesh) for the stereoselective preparation of 8α,12-oxido-13,14,15,16-tetranorlabdane from 8α,12-dihydroxy-13,14,15,16-tetranorlabdane.

## Revendications

1. Procédé de préparation stéréosélective de 8α,12-oxydo-13,14,15,16-tétranorlabdane par déshydratation de 8α,12-dihydroxy-13,14,15,16-tétranorlabdane avec de l'oxyde d'aluminium chargé avec de l'acide, caractérisé en ce que l'on déshydrate le 8α,12-hydroxy-13,14,15,16-tétranorlabdane avec - rapporté à ce 8α,12-dihydroxy-13,14,15,16-tétranorlabdane- 60 à 80 % en poids d'oxyde d'aluminium avec une charge en acide chlorhydrique comprise entre 0,4 et 0,6 % en poids - rapportée à l'oxyde d'aluminium- une densité apparente de 0,9 g/cm$^3$ et un domaine granulométrique compris entre 0,05 et 0,2 mm (70 à 290 mailles).

2. Procédé selon la revendication 1, caractérisé en ce que l'on déshydrate le 8α,12-dihydroxy,13,14,15,16-tétranorlabdane avec - rapporté à ce 8α,12-dihydroxy-13,14,15,16-tétranorlabdane- 70 à 80 % en poids d'oxyde d'aluminium chargé d'acide chlorhydrique.

3. Procédé selon l'une ou les deux revendications 1 à 2, caractérisé en ce que l'on effectue la déshydratation dans des solvants - de préférence dans le toluène et/ou le xylène.

4. Procédé selon l'une ou plusieurs des revendications 1 à 3, caractérisé en ce que l'on déshydrate à des températures comprises entre 120 et 140 °C.

**5.** Utilisation de l'oxyde d'aluminium ayant une charge d'acide chlorhydrique comprise entre 0,4 et 0,6 % en poids - rapporté à l'oxyde d'aluminium -, une densité apparente de 0,9 g/$cm^3$ et un domaine granulométrique compris entre 0,05 et 0,2 mm (70 à 290 mailles) en vue de la préparation stéréosélective du 8α,12-oxydo-13,14,15,16-tétranorlabdane à partir de 8α,12-dihydroxy-13,14,15,16-tétranorlabdane.